# EUROPEAN PATENT APPLICATION

(11) **EP 3 869 518 A1**
(43) Date of publication of application: **25.08.2021**
(21) Application number: 19874239.7
(22) Date of filing: 14.10.2019
(51) Int. Cl.: G16H 20/60

(54) **HEALTH MANAGEMENT SYSTEM**

(30) Priority: 15.10.2018 JP 2018194703
(71) Applicant: Wellnas.Co.,Ltd, Tokyo 105-0001 (JP); Shinshu University, Matsumoto City Nagano 390-8621 (JP)
(72) Inventor: NAKAMURA, Kozo, Kamiina-County Nagano 3994598 (JP)
(74) Representative: Winter, Brandl - Partnerschaft mbB
(86) International application number: PCT/JP2019/040353
(87) International publication number: WO 2020/080320

(57) **Abstract**

To make it possible to manage health in consideration of the individual difference.

A health management system according to the present invention includes a vital data acquisition unit configured to acquire vital data of a user, a dietary ingredient acquisition unit configured to acquire a dietary ingredient that the user ingests, an analysis processing unit configured to analyze a relationship between an intake of the dietary ingredient and improvement or deterioration of the vital data, and a dietary ingredient output unit configured to output the dietary ingredient of improving the vital data and the dietary ingredient of deteriorating the vital data.

## Description

### Technical Field

The present invention relates to a health management system.

### Background Art

Advice is offered to improve dietary lifestyle. For example, Patent Literature 1 discloses a system that analyzes dietary information or vital data to offer advice.

### Citation List

### Patent Literature

Patent Literature 1: JP 2004-302498 A

### Summary of Invention

### Technical Problem

However, the system disclosed in Patent Literature 1 necessitates the preparation of advice in the form of a database in advance, so making it difficult to consider the individual difference.

The present invention is made in view of such a situation and is intended to provide a technology capable of managing health considering the individual difference.

### Solution to Problem

A primary invention of the present invention to solve the above problem is a health management system including a vital data acquisition unit configured to acquire vital data of a user, a dietary ingredient acquisition unit configured to acquire a dietary ingredient that the user ingests, an analysis processing unit configured to analyze a relationship between an intake of the dietary ingredient and improvement or deterioration of the vital data, and a dietary ingredient output unit configured to output the dietary ingredient of improving the vital data and the dietary ingredient of deteriorating the vital data.

The above and other problems and solutions thereto disclosed herein will become apparent from preferred embodiments and drawings of the invention.

### Advantageous Effects of Invention

According to the present invention, it is possible to manage health in consideration of the individual difference.

### Brief Description of Drawings

Fig. 1 is a diagram illustrating an overall configuration example of a health management system according to an embodiment of the present invention.
Fig. 2 is a diagram illustrating a hardware configuration example of user equipment 1.
Fig. 3 is a diagram illustrating a software configuration example of user equipment 1.
Fig. 4 is a diagram illustrating a hardware configuration example of a recommendation apparatus 2.
Fig. 5 is a diagram illustrating a software configuration example of the recommendation apparatus 2 according to the first embodiment.
Fig. 6 is a diagram illustrating a configuration example of a vital data storage unit 231.
Fig. 7 is a diagram illustrating a configuration example of a dietary ingredient storage unit 232.
Fig. 8 is a diagram illustrating processing executed in a health management system according to the first embodiment.
Fig. 9 is a diagram illustrating an example of dietary information displayed on the user equipment 1.
Fig. 10 is a diagram illustrating a software configuration example of a recommendation apparatus 2 according to the second embodiment.
Fig. 11 is a diagram illustrating a configuration example of a dietary reference intake storage unit 234.
Fig. 12 is a diagram illustrating processing executed in a health management system according to the second embodiment.
Fig. 13 is a diagram illustrating an example of dietary information displayed on the user equipment 1 in the second embodiment.
Fig. 14 is a diagram illustrated to describe a target intake.
Fig. 15 is a diagram illustrating a software configuration example of a recommendation apparatus 2 according to the third embodiment.
Fig. 16 is a diagram illustrating a configuration example of a functional food information storage unit 235.
Fig. 17 is a diagram illustrating processing executed in a health management system according to the third embodiment.
Fig. 18 is a diagram illustrating an example of dietary information displayed on the user equipment 1 in the third embodiment.
Fig. 19 is an image diagram of personal dietary optimization according to the present invention.
Fig. 20 is an image illustrating the calculation of an optimal personal diet using a test subject 5 in Fig. 19 as an example.
Fig. 21 is an image diagram of steps for achieving an extension of healthy life expectancy according to the present invention.
Fig. 22 is another image diagram of steps for achieving the extension of healthy life expectancy according to the present invention.
Fig. 23 is a general view illustrating a procedure for determining an optimal diet according to the present invention.
Fig. 24 is a diagram illustrating details of the procedure of Fig. 23.
Fig. 25 is another diagram illustrating details of the procedure of Fig. 23.
Fig. 26 is yet another diagram illustrating details of the procedure of Fig. 23.
Fig. 27 is a general view illustrating a procedure of creating a menu from a difference between reference dietary information and optimal dietary information according to the present invention.
Fig. 28 is a general view illustrating a procedure of creating a menu from only the optimal dietary information according to the present invention.

### Description of Embodiments

Illustrative embodiments of the present invention are in detail described. A health management system according to an embodiment of the present invention has a configuration as below.

[Item 1]
   A health management system including:
   a vital data acquisition unit configured to acquire vital data of a user;
   a dietary ingredient acquisition unit configured to acquire a dietary ingredient that the user ingests;
   an analysis processing unit configured to analyze a relationship between an intake of the dietary ingredient and improvement or deterioration of the vital data; and
   a dietary ingredient output unit configured to output the dietary ingredient of improving the vital data and the dietary ingredient of deteriorating the vital data.
[Item 2]
   The health management system according to Item 1, in which
   the analysis processing unit estimates, in a statistical model using a measured value of the vital data as a response variable and the intake of the dietary ingredient as an explanatory variable, a coefficient of the explanatory variable by multivariate analysis.
[Item 3]
   The health management system according to Item 2, further including:
   a target value input unit configured to receive an input of a target value of the vital data; and
   an intake determination unit configured to determine the intake of the dietary ingredient in such a way that the vital data matches the target value.
[Item 4]
   The health management system according to Item 3, further including:
   a reference value storage unit configured to store a reference value of the intake,
   in which the intake determination unit determines the intake in such a way that a deviation value from the reference value falls in a predetermined range.
[Item 5]
   The health management system according to any one of Items 1 to 4,
   in which
   the intake determination unit determines the intake of the dietary ingredient for a plurality of the users in such a way that the vital data is closest to the target value.
[Item 6]
   The health management system according to any one of Items 1 to 5,
   further including:
   a menu information storage unit configured to store menu information; and
   a menu creation unit configured to determine a menu including the dietary ingredient of the determined intake by referring to the menu information.
[Item 7]
   The health management system according to any one of Items 1 to 5,
   further including:
   a supplementary food recommendation unit configured to recommend a supplementary food including the dietary ingredient that improves the vital data in a case where the dietary ingredient is not ingested by the user.
[Item 8]
   The health management system according to any one of Items 1 to 6,
   further including:
   a menu information acquisition unit configured to acquire the menu information input from the user,
   in which the dietary ingredient acquisition unit specifies and acquires the dietary ingredient from the menu information.
[Item 9]
   The health management system according to Item 7,
   in which
   the menu information includes at least foodstuff information and quantity information.
[Item 10]
   The health management system according to any one of Items 1 to 8,
   in which
   the vital data is a result measured by a blood pressure monitor.
[Item 11]
   The health management system according to any one of Items 1 to 9,
   further including:
   a lifestyle information input unit configured to receive an input of lifestyle information indicating a lifestyle of the user,
   in which the analysis processing unit analyzes a relationship between the lifestyle and the intake of the dietary ingredient and improvement or deterioration of the vital data.

### <First Embodiment>

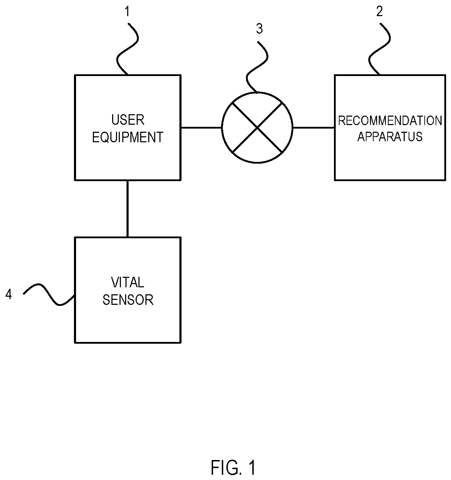

Fig. 1 is a diagram illustrating an overall configuration example of a health management system according to the first embodiment of the present invention. As illustrated in this figure, the health management system according to the first embodiment includes user equipment 1 and a recommendation apparatus 2. The user equipment 1 and the recommendation apparatus 2 are connected to enable communication between them via a communication network 3. The communication network 3 is, for example, the Internet and is constructed as Ethernet (registered trademark), a public switched telephone network, a leased line telephone network, a cellular network, a wireless communication channel, or the like.

The user equipment 1 is a computer operated by a user who is a subject of health management. The user equipment 1 is, for example, a smartphone, a tablet computer, a personal computer, or the like. The user equipment 1 is connected to a vital sensor 4 in such a way that they can communicate with each other. The user equipment 1 and the vital sensor 4 communicate each other using, for example, Bluetooth low energy (BLE) (Bluetooth is a registered trademark) or serial communication. In the present embodiment, the vital sensor 4 is assumed to be a blood pressure monitor.

The recommendation apparatus 2 is a computer that provides the user with information regarding diet. The recommendation apparatus 2 is, for example, a personal computer or a workstation. The recommendation apparatus 2 can also be configured as a cloud computing-based virtual computer.

The health management system according to the present embodiment offers a recommendation regarding diet depending on both the dietary ingredient such as nutrients ingested by the user and the user's vital data. In the first embodiment, the vital data, particularly dietary ingredients that affect the stabilization or elevation of systolic blood pressure, can be specified and output, allowing it to be referenced upon considering a menu of meal. The vital data according to the present embodiment can include, but is not limited to, height, weight, BMI, body fat percentage, muscle mass, bone density, blood pressure, pulse, pulse wave, body temperature, electrocardiogram, blood oxygen level, respiratory rate, or the like.

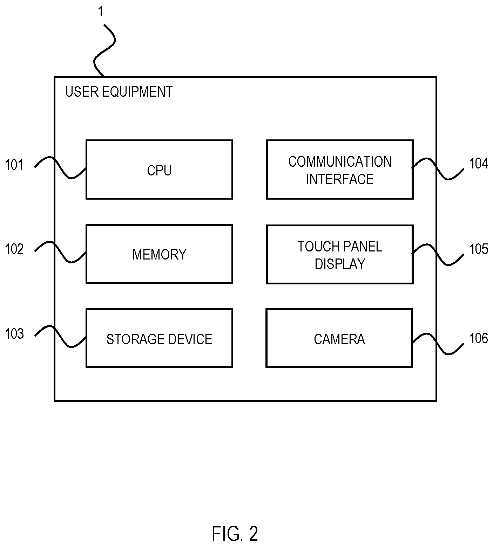

Fig. 2 is a diagram illustrating a hardware configuration example of user equipment 1. The user equipment 1 includes a CPU 101, a memory 102, a storage device 103, a communication interface 104, a touch panel display 105, and a camera 106. The storage device 103 is, for example, a hard disk drive, a solid-state drive, a flash memory, or the like, which stores various data or programs. The communication interface 104 is an interface used for the connection to the communication network 3.

The communication interface 104 is, for example, an adapter used for connecting to Ethernet (registered trademark), a modem used for connecting to a public switched telephone network, a transceiver used for wireless communication, a universal serial bus (USB) connector or RS232C connector used for serial communication, or the like. The touch panel display 105 is a device that inputs and outputs data. The camera 106 generates image data of a target to be captured.

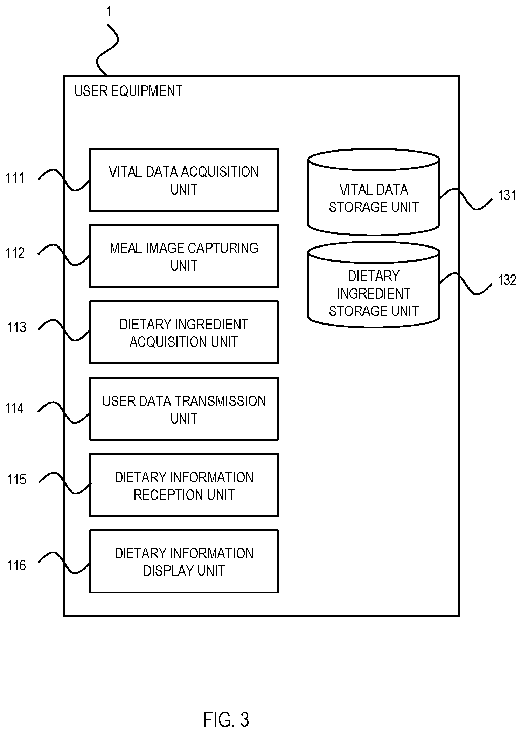

Fig. 3 is a diagram illustrating a software configuration example of user equipment 1. The user equipment 1 includes a vital data acquisition unit 111, a meal image capturing unit 112, a dietary ingredient acquisition unit 113, a user data transmission unit 114, a dietary information reception unit 115, a dietary information display unit 116, a vital data storage unit 131, and a dietary ingredient storage unit 132.

The vital data acquisition unit 111 acquires vital data from the vital sensor 4. In the present embodiment, the vital sensor 4 is assumed to be a blood pressure monitor, and the vital data acquisition unit 111 acquires systolic blood pressure and diastolic blood pressure. Moreover, a plurality of vital sensors 4 can acquire a plurality of vital data types. The vital data acquisition unit 111 registers the vital data in the vital data storage unit 131. The vital data storage unit 131 is capable of storing the vital data in association with the date and time when the vital data is measured by the vital sensor 4 (or the date and time of receiving by the vital data acquisition unit 111 the vital data from the vital sensor 4), information used to specify the user (hereinafter referred to as a user ID) and information used to identify the type of vital data (hereinafter referred to as vital data ID).

The meal image capturing unit 112 captures an image of the meal ingested by the user (hereinafter referred to as a meal image). The meal image capturing unit 112 can activate the camera 106 by the user's operation to acquire the meal image.

The dietary ingredient acquisition unit 113 acquires the amount of dietary ingredients such as nutrient and energy ingested by the user on the basis of the meal image. For example, the dietary ingredient acquisition unit 113 is capable of transmitting the meal image to an external expert such as a dietitian for causing the external expert to input a dietary ingredient and its content, and capable of receiving them from the external expert. In addition, the dietary ingredient acquisition unit 113 can learn the dietary ingredient amount included in the image by machine learning or the like and can estimate the content of the dietary ingredient by performing image analysis on the meal image. Moreover, the dietary ingredient acquisition unit 113 can receive the input of ingested nutrients or the like by the user without using the image analysis. The dietary ingredient acquisition unit 113 registers the intake of the acquired dietary ingredient in the dietary ingredient storage unit 132. In the dietary ingredient storage unit 132, the user ID, the information used to specify identifies the dietary ingredient (hereinafter referred to as a dietary ingredient ID), and the intake of the dietary ingredient can be recorded in association with the date and time of ingesting foods by the user.

The user data transmission unit 114 transmits information including the user's vital data and the intake of dietary ingredients ingested by the user (hereinafter referred to as user data) to the recommendation apparatus 2. The user data transmission unit 114 can read out the vital data and the intake of the dietary ingredient for the previous day from the vital data storage unit 131 and the dietary ingredient storage unit 132, for example, once a day, and can transmit the read ones as user data to the recommendation apparatus 2. In addition, the user data transmission unit 114 can transmit the user data including only the vital data at every acquisition of the vital data to the recommendation apparatus 2 or can transmit the user data including only the intake of dietary ingredient at every acquisition of the dietary ingredients to the recommendation apparatus 2.

The dietary information reception unit 115 receives the information relating to the user's diet (hereinafter referred to as dietary information) transmitted from the recommendation apparatus 2. The dietary information display unit 116 displays the dietary information.

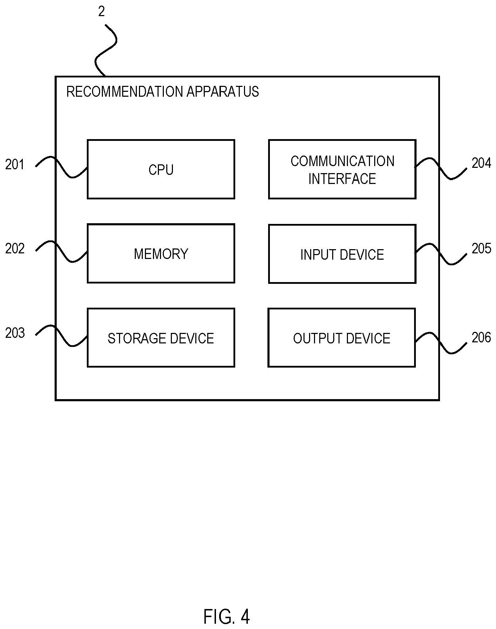

Fig. 4 is a diagram illustrating a hardware configuration example of a recommendation apparatus 2. As illustrated in this figure, the recommendation apparatus 2 includes a CPU 201, a memory 202, a storage device 203, a communication interface 204, an input device 205, and an output device 206. The storage device 203 is, for example, a hard disk drive, a solid-state drive, a flash memory, or the like, which stores various data or programs. The communication interface 204 is an interface used for the connection to the communication network 3. The communication interface 204 is, for example, an adapter used for connecting to Ethernet (registered trademark), a modem used for connecting to a public switched telephone network, a transceiver used for wireless communication, a USB connector or RS232C connector used for serial communication, or the like. The input device 205 is, for example, a keyboard, a mouse, a touch panel, a button, a microphone, or the like that is used for inputting data. The output device 206 is, for example, a display, a printer, a speaker, or the like that is used for outputting data.

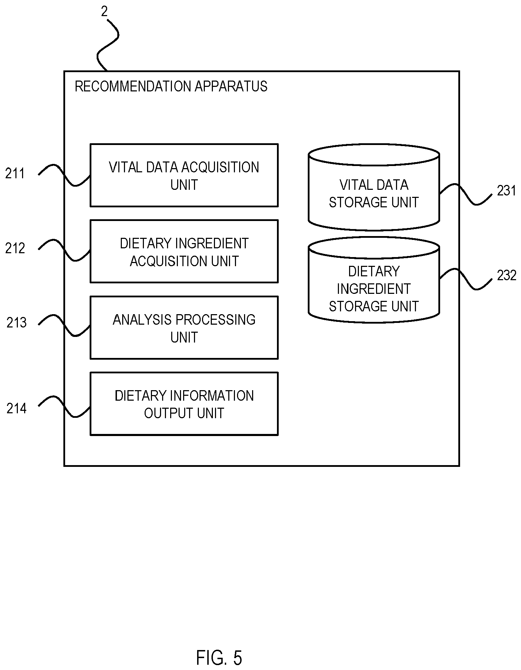

Fig. 5 is a diagram illustrating a software configuration example of the recommendation apparatus 2 according to the first embodiment. As illustrated in this figure, the recommendation apparatus 2 includes a vital data acquisition unit 211, a dietary ingredient acquisition unit 212, an analysis processing unit 213, a dietary information output unit 214, a vital data storage unit 231, and a dietary ingredient storage unit 232.

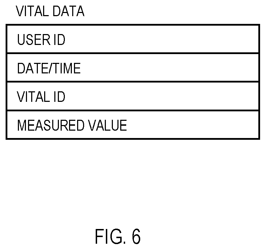

The vital data acquisition unit 211 acquires the user's vital data. In the present embodiment, the vital data acquisition unit 211 acquires the vital data from the user data transmitted by the user equipment 1. The vital data acquisition unit 211 registers the acquired vital data in the vital data storage unit 231. Fig. 6 is a diagram illustrating a configuration example of a vital data storage unit 231. The vital data storage unit 231 stores the date and time of measuring the vital data, the vital data ID indicating the type of the vital data, and a measured value of the vital data, in association with the user ID used for identifying the user.

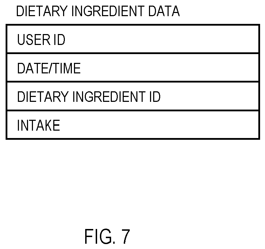

The dietary ingredient acquisition unit 212 acquires the dietary ingredient ingested by the user. In the present embodiment, the dietary ingredient acquisition unit 212 acquires the dietary ingredient from the user data transmitted from the user equipment 1. The dietary ingredient acquisition unit 212 registers the acquired dietary ingredient in the dietary ingredient storage unit 232. Fig. 7 is a diagram illustrating a configuration example of a dietary ingredient storage unit 232. The dietary ingredient storage unit 232 stores a user ID, date and time when the user ingests foods, a dietary ingredient ID used for identifying dietary ingredients, and the intake of dietary ingredients in association with among them.

The analysis processing unit 213 analyzes a relationship between the dietary ingredient and the vital data. In the first embodiment, the analysis processing unit 213 is capable of performing the single regression analysis on the vital data (such as systolic and diastolic blood pressure) for each of various dietary ingredients such as intake energy, protein, fat, carbohydrate, and vitamin A.

The dietary information output unit 214 outputs the information relating to the user's diet (hereinafter referred to as dietary information). In the first embodiment, the dietary information output unit 214 is capable of transmitting a single correlation coefficient of each dietary ingredient to the user equipment 1 as the dietary information. The dietary information output unit 214 sets a dietary ingredient having one of positive or negative correlation as a dietary ingredient that improves the vital data depending on the type of vital data. The dietary information output unit 214 sets a dietary ingredient having the other one of the positive or negative correlation as a dietary ingredient that deteriorates the vital data depending on the type of vital data. Thus, it is possible to distinguish and output the dietary ingredients that improve or deteriorate the vital data.

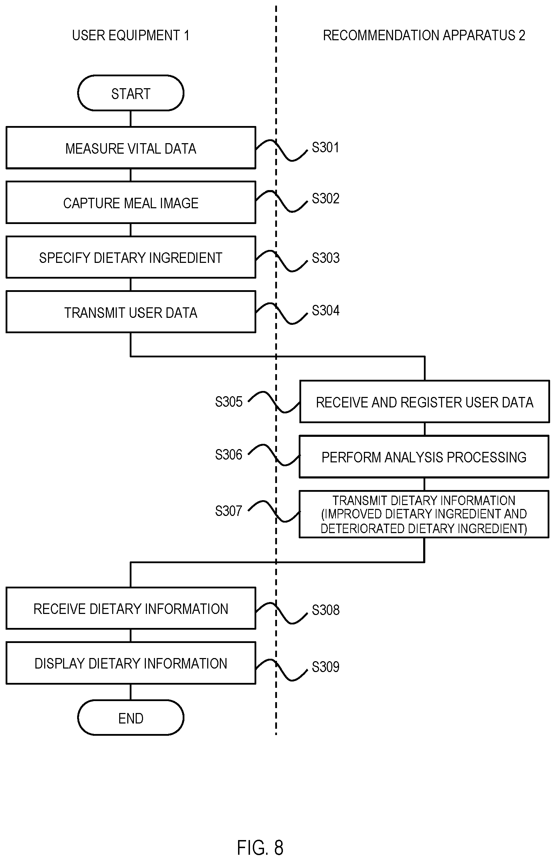

Fig. 8 is a diagram illustrating processing executed in a health management system according to the first embodiment. In the user equipment 1, the vital data acquisition unit 111 acquires the vital data from the vital sensor 4 (S301). The meal image capturing unit 112 controls the camera 106 to capture a meal image (S302), and the dietary ingredient acquisition unit 113 analyzes the captured meal image to specify the content of dietary ingredients contained in the diet (S303). The user data transmission unit 114 transmits user data including the vital data and the content of dietary ingredients (S304).

When the recommendation apparatus 2 receives the user data, the vital data acquisition unit 211 acquires the vital data from the user data, and the dietary ingredient acquisition unit 212 acquires the intake of the dietary ingredient from the user data. The analysis processing unit 213 analyzes the correlation between each dietary ingredient and the vital data (S306). The dietary information output unit 214 transmits the dietary information for each dietary ingredient to the user equipment 1 (S307). The dietary information includes the dietary ingredient that has a positive or zero correlation coefficient and improves the vital data (improving dietary ingredient) and the dietary ingredient that has a negative correlation coefficient and deteriorates the vital data (deteriorating dietary ingredient). Moreover, for example, depending on the vital data, in a case where the body weight or blood pressure exceeds an appropriate value, the explanatory variable with a positive correlation coefficient is set as a deterioration factor, and the explanatory variable with a negative correlation coefficient is set as an improvement factor. Thus, in defining improvement and deterioration, it is necessary to be considered whether each vital data of the user exceeds or falls down the appropriate value.

In the user equipment 1, the dietary information reception unit 115 receives the dietary information transmitted from the recommendation apparatus (S308), and the dietary information display unit 116 displays the received dietary information on the touch panel display 105 (S309).

As described above, the dietary ingredient that improves or deteriorates the user's vital data is output to the user equipment 1, so it can be used as a reference upon preparing a meal for the user.

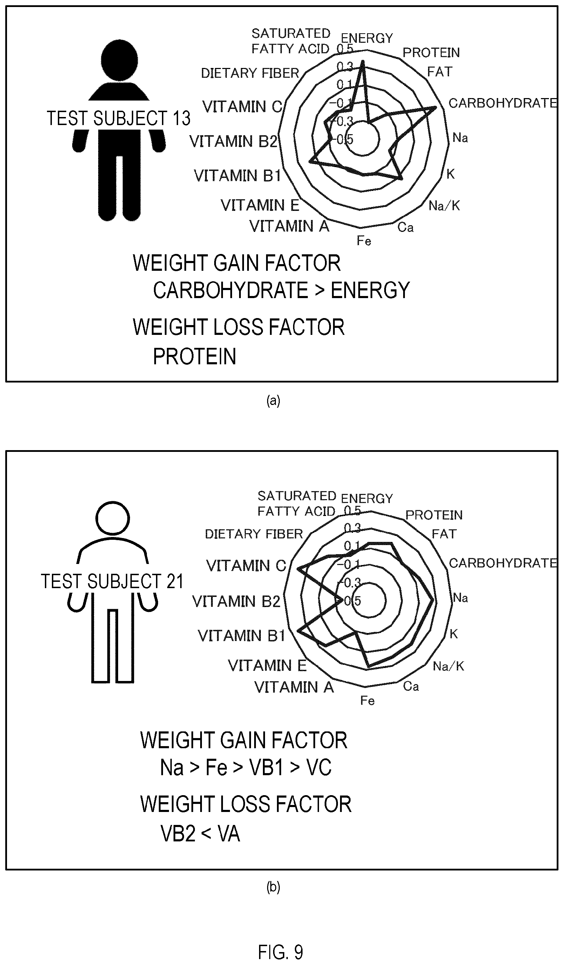

Fig. 9 is a diagram illustrating an example of dietary information displayed on the user equipment 1. As illustrated in Fig. 9(a), it can be seen that a test subject 13 gains weight upon ingesting carbohydrates and energy with the correlation coefficient shown in the figure but loses weight upon ingesting proteins. On the other hand, as illustrated in Fig. 9(b), it can be seen that a test subject 21 gains weight upon ingesting sodium, iron, vitamin B1, vitamin C, or the like but loses weight upon ingesting vitamin B2, vitamin A, or the like. In this way, each individual has different dietary ingredients involved in weight. The diet that decreases the dietary ingredients with a larger positive correlation coefficient and increases the dietary ingredients with a smaller negative correlation coefficient makes it possible to reduce efficiently the weight. The dietary ingredients exert different effects depending on the users, so the health management system of the present embodiment is capable of displaying which dietary ingredient affects the user's vital data on the user equipment 1.

### <Second Embodiment>

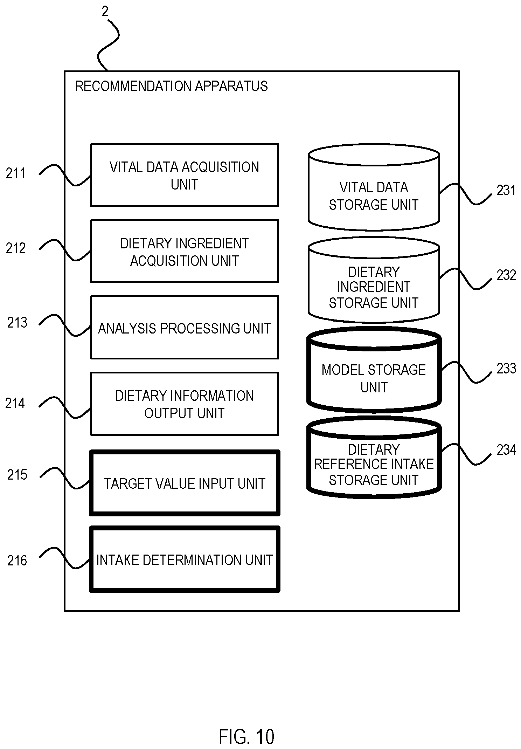

In the first embodiment, the dietary ingredient based on the simple regression coefficient for the vital data used as the dietary information is displayed, but in the second embodiment, multivariate analysis (multiple regression analysis) is performed by setting the vital data as the dietary information and setting the dietary ingredient as an explanatory variable. Besides, in the second embodiment, a coefficient of each dietary ingredient is output, a target value of the vital data is received, and the intake of dietary ingredient such that the vital data can be the target value is displayed. Fig. 10 is a diagram illustrating a software configuration example of a recommendation apparatus 2 according to the second embodiment. The recommendation apparatus 2 of the second embodiment includes a target value input unit 215, an intake determination unit 216, a model storage unit 233, and a dietary reference intake storage unit 234, in addition to each functional unit and storage unit included in the recommendation apparatus 2 of the first embodiment. The multivariate analysis according to the present embodiment includes simple regression analysis, multiple regression analysis, principal component analysis, independent component analysis, factor analysis, quantification theory (types I, II, III, and IV), cluster analysis, conjoint analysis, and multidimensional scaling (MDS).

The model storage unit 233 stores a statistical model in which the vital data is used as the response variable and the dietary ingredient is used as the explanatory variable. In addition, parameters such as a regression coefficient and a constant applied to the statistical model are also stored in the model storage unit 233.

In the second embodiment, the analysis processing unit 213 applies the vital data relating to the user and the intake of the dietary ingredient to the statistical model for each user to perform the multiple regression analysis and to estimate the regression coefficient and constant of each dietary ingredient. The analysis processing unit 213 registers the estimated regression coefficient and constant in the model storage unit 233 in association with the user ID. Applying the dietary ingredient to the statistical model to which an estimated regression coefficient and constant are applied (hereinafter referred to as an estimation model) makes it possible to estimate the user's vital data in the case where the user ingests a meal including such dietary ingredients.

The target value input unit 215 receives the input of a target value of the vital data. For example, the target value input unit 215 can receive the target value that is input by the user on the user equipment 1 or can receive the input of the target value from the input device 205 such as a keyboard.

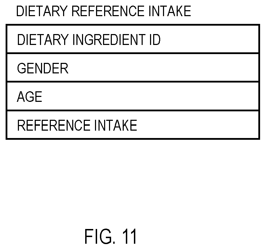

The dietary reference intake storage unit 234 stores the energy and nutrients reference intakes *(Dietary Reference Intakes for Japanese),* which are released by the Ministry of Health, Labour and Welfare and are referenced to maintain and promote their health and to prevent the progression of lifestyle-related diseases for Japanese. Moreover, the dietary reference intake storage unit 234 can store an optional reference value instead of the one released by the Ministry of Health, Labour and Welfare. Fig. 11 is a diagram illustrating a configuration example of a dietary reference intake storage unit 234. As illustrated in this figure, the dietary reference intake storage unit 234 stores a reference intake value of the dietary ingredient in association with the dietary ingredient ID indicating the dietary ingredient, gender, and age. Moreover, the dietary reference intake storage unit 234 can store the reference value in association with the user's attribute such as body weight and BMI in place of or in addition to at least one of gender or age.

The intake determination unit 216 determines an amount of each dietary ingredient (hereinafter referred to as a target intake) so that the vital data of a result calculated by the estimation model matches or is closest to the target value received by the target value input unit 215. For example, for each dietary ingredient, the intake determination unit 216 reads out the reference value corresponding to the user's attribute from the dietary reference intake storage unit 234 and applies it to the estimation model. If the applied result is larger than the target value, the intake determination unit 216 decreases the amount of the dietary ingredient with the positive regression coefficient and increases the amount of the dietary ingredient with a negative regression coefficient, and repeats the calculation so that the vital data can approach the target value. Thus, it is possible to determine the target intake for each dietary ingredient. The amount to increase or decrease the dietary ingredient can be, for example, a predetermined ratio of the dietary ingredient (e.g., an optional value such as 1%). Moreover, the intake determination unit 216 is capable of using any algorithm as long as the target intake of each dietary ingredient can be determined so that the vital data is closest to the target value.

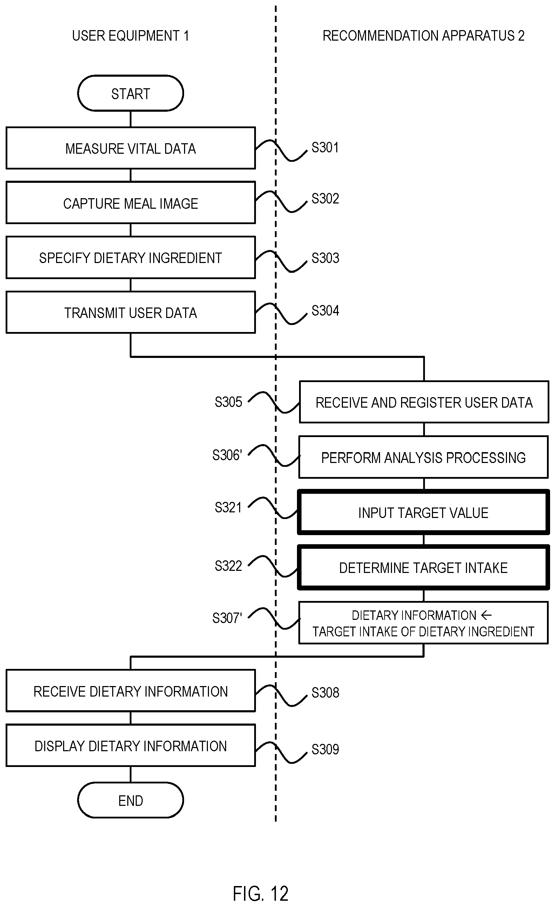

Fig. 12 is a diagram illustrating processing executed in a health management system according to the second embodiment. In the processing of Fig. 12, comparing to the processing illustrated in Fig. 8, instead of step S306, the analysis processing unit 213 applies the vital data and the dietary ingredient intake regarding the user who transmitted the user data to the statistical model and performs multiple regression analysis as described above to estimate the regression coefficient and constant of each dietary ingredient (S306'). In addition, following step S306', the target value input unit 215 receives the input of the target value of the vital data (S321). The intake determination unit 216 determines the target intake of each dietary ingredient so that the vital data can be closest to the target value (S322). Furthermore, instead of step S307, the dietary information output unit 214 transmits the target intake as the dietary information to the user equipment 1 (S307').

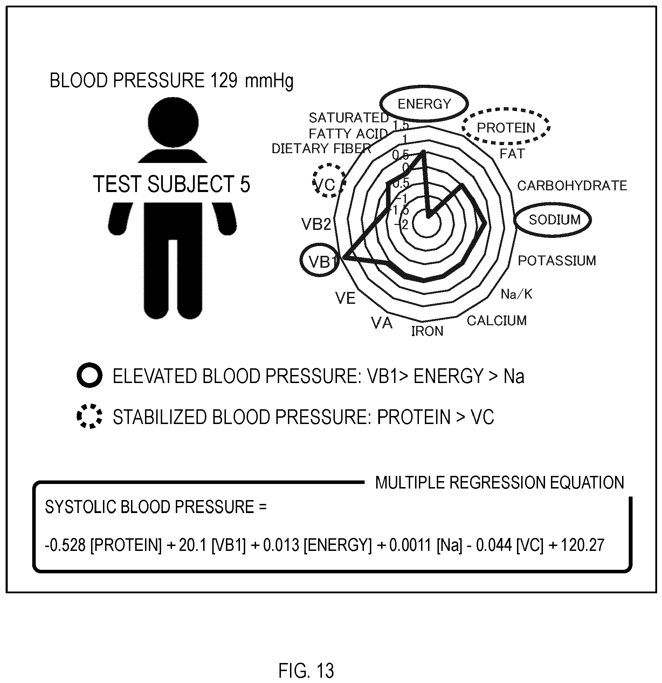

Fig. 13 is a diagram illustrating an example of dietary information displayed on the user equipment 1 in the second embodiment. In the example of Fig. 13, a multiple regression equation relating to the systolic blood pressure as vital data is illustrated. By referring to this, it is possible to easily know what kind of dietary ingredient affects the systolic blood pressure of the user as a test subject 5.

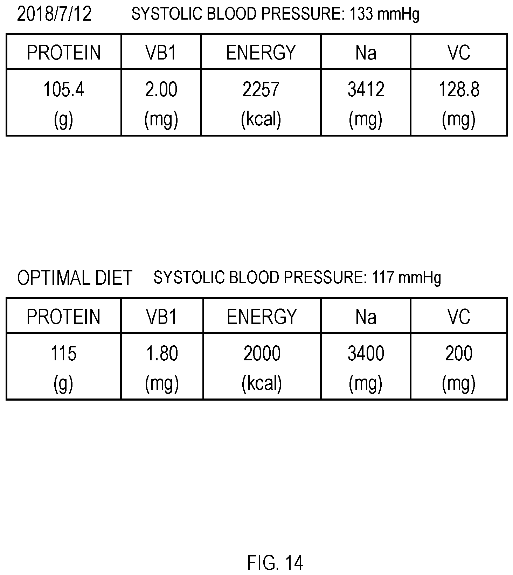

Fig. 14 is a diagram illustrated to describe a target intake. In the example of Fig. 14, the systolic blood pressure measured on July 12, 2018 was 133 mmHg, and the ingested dietary ingredients were listed in the upper table. When the target value of the systolic blood pressure was 118 mmHg and the dietary ingredients were determined, the predicted value of the vital data was 117 mmHg, and the target intakes of the dietary ingredients were as listed in the lower table. In this way, it is possible to indicate the dietary ingredients necessary to be included in the diet (optimal diet) that can achieve the target value of the vital data, so it is possible to easily plan a menu of the optimal diet with reference to the dietary ingredients.

### <Third Embodiment>

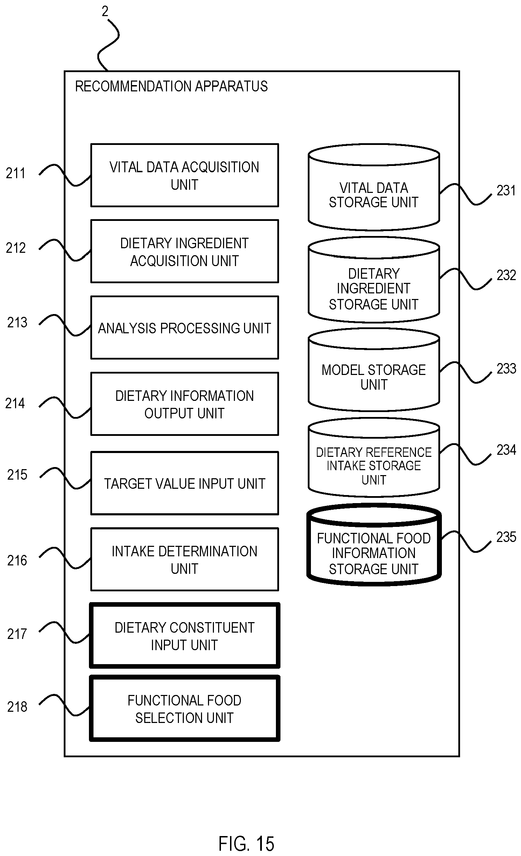

In the third embodiment, a recommendation is offered that combines the dietary information with a supplementary food. The supplementary food in the present embodiment is offered as a recommendation in combination with one or both of a functional food containing a nutritional component and a functional food containing a non-nutrient component. In the following description, foods containing a nutritional component or a non-nutrient component are collectively referred to as "functional food". Moreover, the nutritional components are nutritional component labeling items based on the Food Labeling Law (calorie, proteins, fats, carbohydrates, sodium, saturated fatty acids, n-3 fatty acids, n-6 fatty acids, cholesterols, carbohydrates, sugars (monosaccharides or disaccharides, limited to non-sugar alcohols), dietary fiber, zinc, potassium, calcium, chromium, selenium, iron, copper, magnesium, manganese, molybdenum, iodine, phosphorus, niacin, pantothenic acid, biotin, vitamins A, B1, B2, B6, B12, C, D, E, and K, and folic acid). The non-nutrient component is, for example, a component other than nutritional components such as polyphenols and GABA, and a component specified among the nutrients as a group composed of a plurality of components (e.g., cyclodextrin as dietary fiber). Fig. 15 is a diagram illustrating a software configuration example of a recommendation apparatus 2 according to the third embodiment. The recommendation apparatus 2 according to the third embodiment includes a dietary constituent input unit 217, a functional food selection unit 218, and a functional food information storage unit 235, in addition to each functional unit and storage unit illustrated in Fig. 10.

The dietary constituent input unit 217 receives as an input the constituent of the diet to be ingested. The dietary constituent includes the dietary ingredients contained in the diet. In addition, the dietary constituent can include a specification or an image for the dietary.

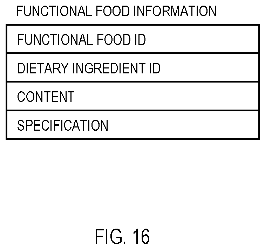

The functional food information storage unit 235 stores information regarding functional foods. Fig. 16 is a diagram illustrating a configuration example of a functional food information storage unit 235. The functional food information storage unit 235 stores the dietary ingredient ID indicating the dietary ingredient contained in the functional food and the content of the dietary ingredient or a specification for the functional foods, in association with the information used for identifying the functional food (a functional food ID)

The functional food selection unit 218 selects a functional food that complements the dietary ingredients that the user lacks. The functional food selection unit 218 is capable of comparing the amount of the dietary ingredient contained in the input dietary constituent with the target intake determined by the intake determination unit 216 described above to select a functional food containing a dietary ingredient that is not reached the target intake from the functional food information storage unit 235. In the case where a plurality of functional foods contains the deficient dietary ingredient as described above, the functional food selection unit 218 is capable of selecting, for example, a functional food that is less than or equal to the shortage and contains the maximum amount. In addition, in the case where a plurality of functional foods is combined, it is possible to select a combination of functional foods that minimize the types of functional foods.

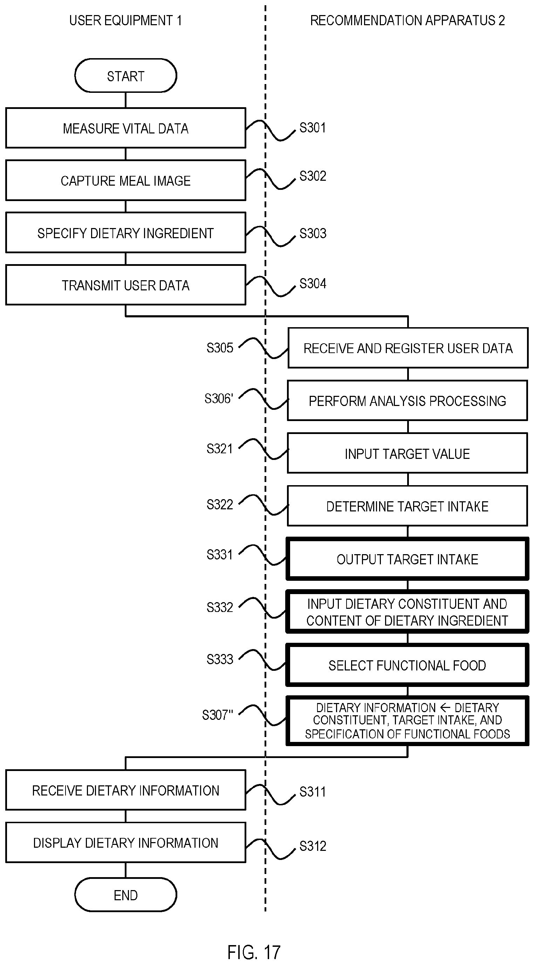

Fig. 17 is a diagram illustrating processing executed in a health management system according to the third embodiment. In the processing illustrated in Fig. 17, compared with the processing shown in Fig. 12, following step S322, the intake determination unit 216 outputs the determined target intake (S331). The intake determination unit 216 can output it to the output device 206 of the recommendation apparatus 2 or can transmit the target intake to the user equipment 1 for causing the target intake to be output to the user equipment 1. Then, the dietary constituent input unit 217 receives the dietary constituent and the amount of the dietary ingredient contained in the diet as an input (S332). The dietary constituent is assumed to be determined with reference to the output target intake. The dietary constituent input unit 217 can receive the input from the input device 205 of the recommendation apparatus 2 or can receive, from the user equipment 1, the dietary constituent and the amount of dietary ingredients input through the user equipment 1. The functional food selection unit 218 specifies a deficient dietary ingredient in which the amount of the input dietary ingredient is less than the target intake of the dietary ingredient relating to the optimal diet. The functional food selection unit 218 selects a functional food containing a deficient dietary ingredient from the functional food information storage unit 235 (S333). The dietary information output unit 214 transmits the dietary information including the input dietary constituent, the target intake, and the specification of the functional food to the user equipment 1 (S307"), instead of step S307'.

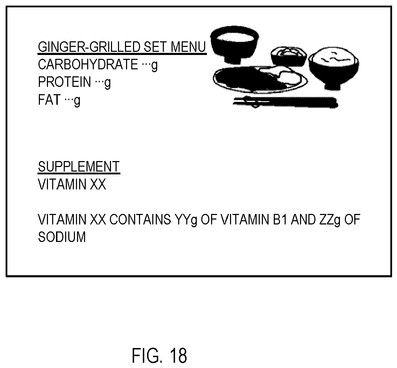

Fig. 18 is a diagram illustrating an example of dietary information displayed on the user equipment 1 in the third embodiment. In the example of Fig. 18, a functional food is recommended together with the dietary constituent. This makes it possible to supplement nutrients and non-nutritive functional ingredients insufficient with diet alone with functional foods (supplements), bringing the vital data closer to the target value.

### <Fourth Embodiment>

In the second and third embodiments described above, the target intake for a single user is determined, but the target intake can be determined for a group of a plurality of users. In the fourth embodiment as well, the target value input unit 215 receives as an input the target value of vital data for each user, and the analysis processing unit 213 also performs the multiple regression analysis for each user to estimate the parameters of the statistical model. However, the intake determination unit 216 applies the same amount of dietary ingredients to all users with respect to the estimation model for each user and determines the target intake so that the sum of differences between the estimated value and the target value of the vital data of each user is minimized. This makes it possible to provide a meal so that the values of vital data approach the target values as a whole in the case of providing a meal of the same menu to each user in the group, for example, in a dining room or the like.

In the fourth embodiment, the analysis unit 213 determines a target intake for each user separately from the above-mentioned target intake to be determined for the group. The dietary constituent input unit 217 outputs the target intake for the group. The functional food selection unit 218 can compare the difference between the target intake for each user and the amount of the dietary ingredient contained in the diet to specify the deficient dietary ingredient and can allow each user to select a functional food that supplements the lack of dietary ingredients. In this case, it is possible to adjust the optimal amount of dietary ingredients for each user depending on the functional food, so the vital data of each user is expected to approach the target value.

### <Fifth Embodiment>

In the first to fourth embodiments described above, only the dietary ingredient is set as the explanatory variable of the estimation model, but the behavior related to the lifestyle can be added to the explanatory variable. In this case, the recommendation apparatus 2 is provided with a lifestyle information input unit that receives the input of information indicating lifestyle such as exercise, drinking, smoking, stress, and sleep. The analysis processing unit 213 can perform the multiple regression analysis by adding information indicating such lifestyle as the explanatory variable. This makes it possible to provide optimal dietary information in consideration of the influence of the combination of the user's lifestyle and dietary ingredients on the user's vital data.

Although the embodiments are described above, the embodiments described above are intended to facilitate the understanding of the present invention, and should not be construed as limitations on the scope of the present invention. The present invention covers all modifications, alterations, and equivalents thereof without departing from the spirit and scope of the invention.

In one example, in the present embodiment, the vital sensor 4 is a device for measuring blood pressure, but a weight scale, a body composition monitor, or the like can be used as the vital sensor. In addition, a device for measuring biochemical blood parameters (such as blood glucose, cholesterol, neutrality, γ-GTP, or HbA1c) can be used. Also in this case, it is preferable to use a non-invasive measuring device.

Further, in the present embodiment, the dietary constituent is input from the recommendation apparatus 2 or the user equipment 1, but the intake determination unit 216 can transmit the target intake to a computer, which is operated by a dietitian, a cook, or the like who thinks about menus, to receive the input from the dietitian or the cook.

Further, in the present embodiment, the correlation between the dietary ingredient and the vital data is analyzed by regression analysis, but it is possible to use machine learning to perform the analysis. For example, it is possible to perform the learning using a neural network by using dietary ingredients as an input signal and vital data as a supervisor signal.

Although the present embodiment has the so-called server-client configuration in which the analysis processing is performed by the recommendation apparatus 2, the user equipment 1 is provided with all the functions of the recommendation apparatus 2, and only the user equipment 1 can recommend the optimal diet.

### <Example 1>

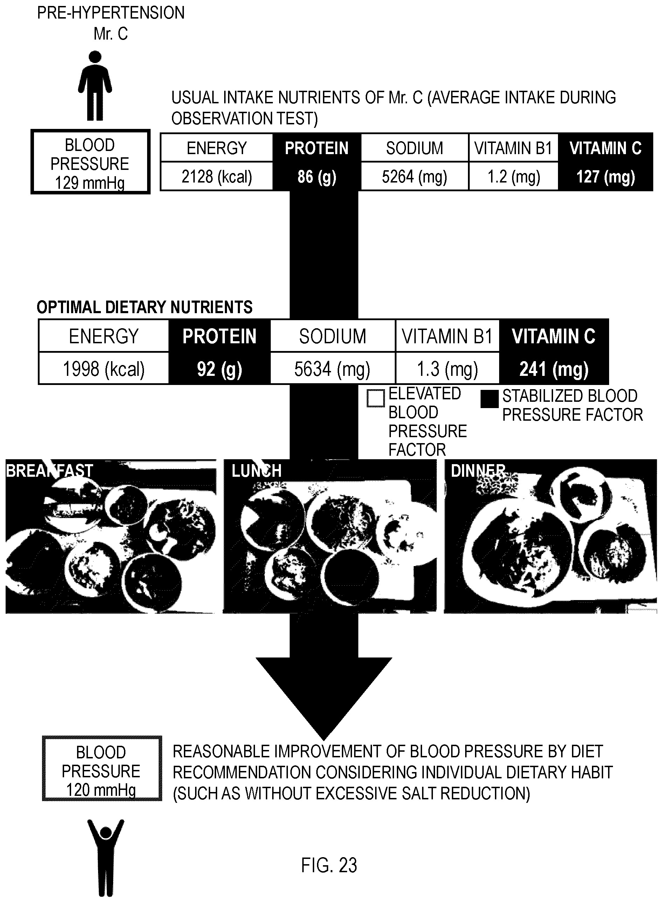

A specific example of the optimal diet using the recommendation apparatus according to the present invention including the first to fourth embodiments described above is now described. In this example, the optimal nutrients calculated by the method according to the present invention described above are compared with the nutrients ingested by the user (average nutrients). In one example, as illustrated in Fig. 23, from the "usual intake nutrients (average intake during observation test)" of the prehypertension "Mr. C (blood pressure: 129 mmHg)", the amount of the elevated blood pressure factor (as illustrated, energy, sodium, and vitamin B1) of Mr. C is reduced. In addition, the amount of the stabilized blood pressure factor (as illustrated, protein and vitamin C) is increased. Thus, the optimal nutrients (per day) are calculated.

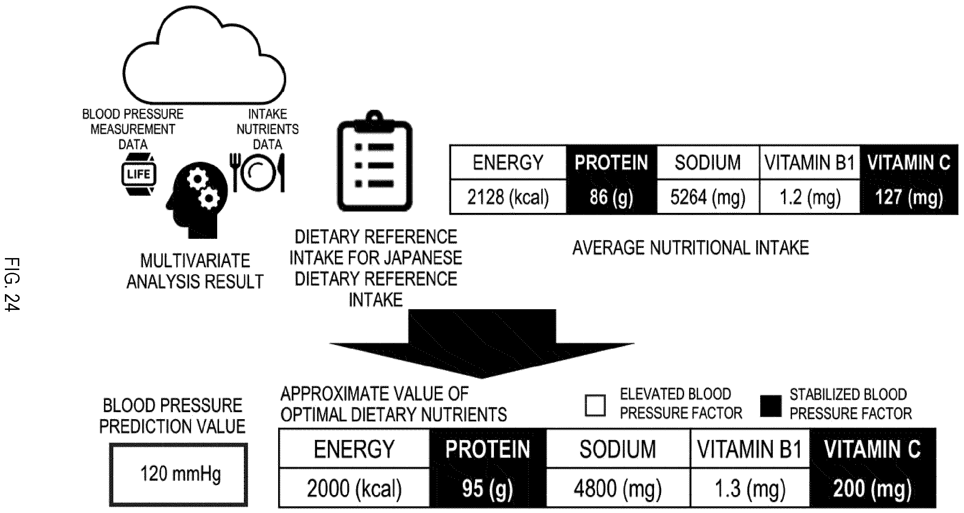

Moreover, as illustrated in Fig. 24, in practice, the multivariate analysis results obtained from the blood pressure and intake nutrient data collected during the observation period, the *Dietary Reference Intakes for Japanese,* and the *average nutrient intakes for individuals* can be referred. Thus, while referring to them, the approximate value of the daily nutrient amount of the optimal diet within a reasonable range for the test subject is determined.

It is possible to obtain this approximate value of optimal dietary nutrients as below. The multiple regression analysis of blood pressure and intake nutrient data acquired for each user is performed. The nutrients that affect blood pressure are specified from energy, protein, fat, carbohydrate, calcium, iron, vitamin A, vitamin E, vitamin B1, vitamin B2, vitamin C, dietary fiber, saturated fatty acids, sodium, and potassium. Then, the relational expression between blood pressure and nutrient intake is obtained. From this relational expression, the amount of nutrients that affect the blood pressure is obtained so that the blood pressure is 10 mmHg lower than the average blood pressure of the user. Then, all the nutrient amounts are specified to satisfy Conditions 1) to 3) below: Condition 1) is that the amount of the three major nutrients (carbohydrates, fat, and proteins) defined in the Dietary Reference Intakes for Japanese (2015 edition) is the amount that is more than or equal to the minimum value and less than 120% of the maximum value in the user's age and gender; Condition 2) is one that does not exceed it for nutrients with a tolerable upper limit; Condition 3) is that the specified amount of blood pressure-related component is 80% or more of the estimated average required amount or the standard amount in the *Dietary Reference Intakes for Japanese.*

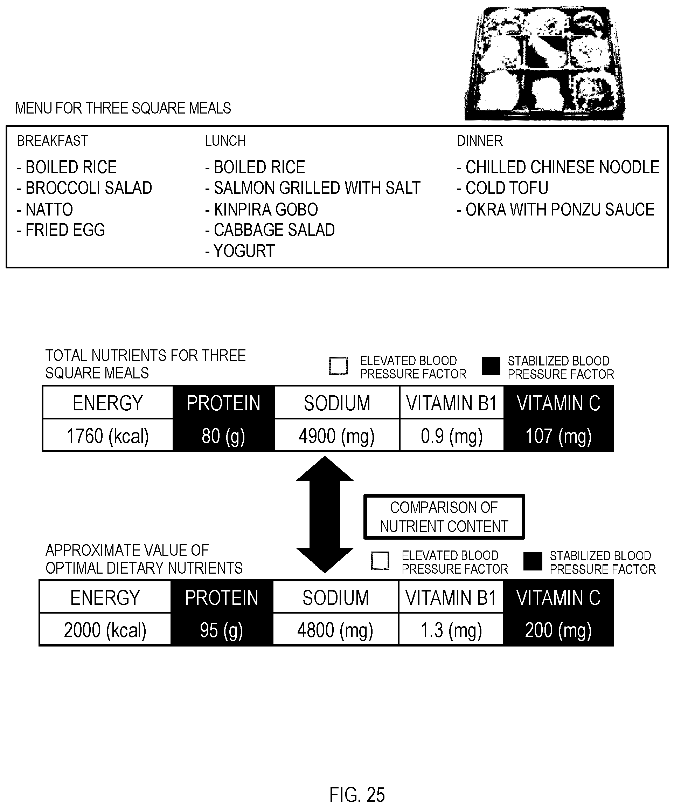

Subsequently, as illustrated in Fig. 25, for example, a lunch box manufacturer, a registered dietitian, or the like prepares menus of breakfast, lunch, and dinner as fundamentals. The total amount of nutrients is specified by calculating and adding up the amounts of nutrients contained in breakfast, lunch, and dinner on the basis of the *Standard Tables of Food Composition in Japan* from the content of foodstuff and seasonings used. Excess or deficiency of nutrients is specified by comparing the amount of this nutrient with the optimal dietary nutrient approximate value obtained earlier for each nutrient.

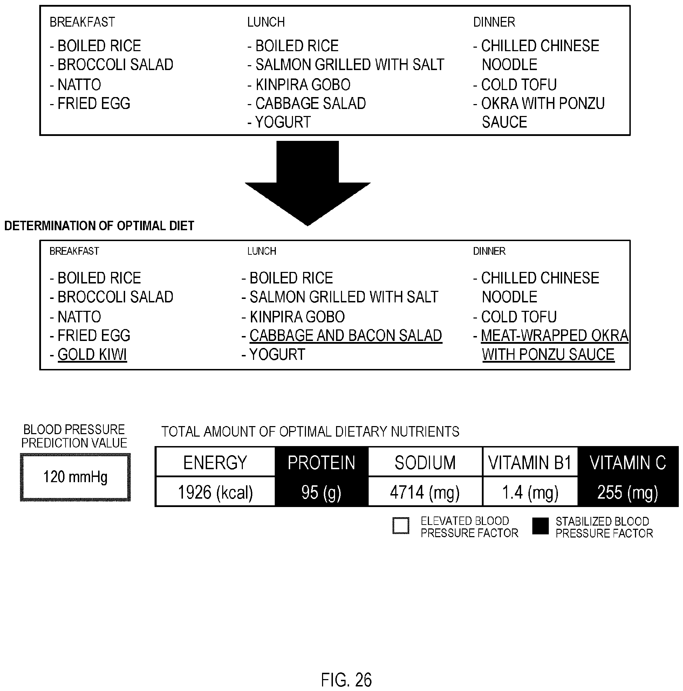

Finally, as illustrated in Fig. 26, the menu is changed so that there is no excess or deficiency from the approximate value of optimal dietary nutrients, and the optimal diet for each user is determined. In the illustrated example, the modified ones are underlined.

### <Example 2>

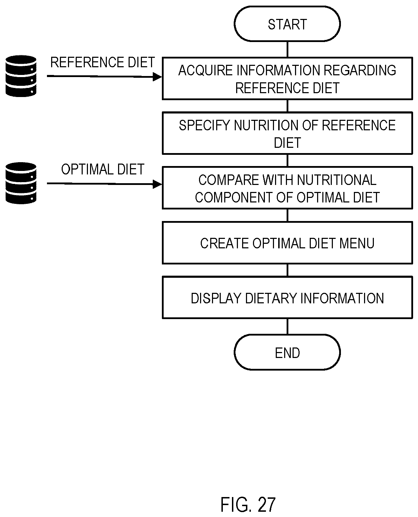

In Example 1 described above, the health management system performs (1) acquisition of information regarding the reference diet (breakfast, lunch, and dinner as fundamentals described above), (2) specifying the nutritional components of the reference diet, (3) comparing with the nutritional components of the optimal diet, and (4) creation of an optimal diet menu based on the comparison result (see Fig. 27).

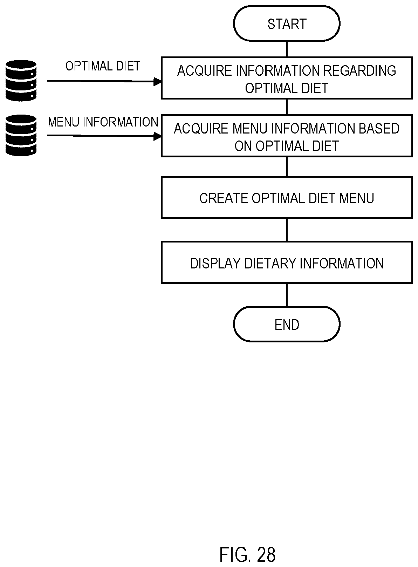

However, the present invention is not limited to the examples described above. For example, the health management system further includes a menu information acquisition unit that stores menu information (nutrients of each menu are associated with each menu). Thus, the health management system can create a menu in only two steps: (1) acquiring nutritional components of the optimal diet and (2) determining a menu by referring to the menu information by the menu information acquisition unit (Fig. 28). In other words, the menu information can be created directly from the information of the optimal diet without acquiring the reference diet (or input from the lunch box manufacturer, the registered dietitian, or the like).

The description of the above-mentioned examples is merely illustrative. For example, the adjustment can be performed by factors other than snacks or foodstuffs (such as supplements or nutritional supplements).

### Industrial Applicability

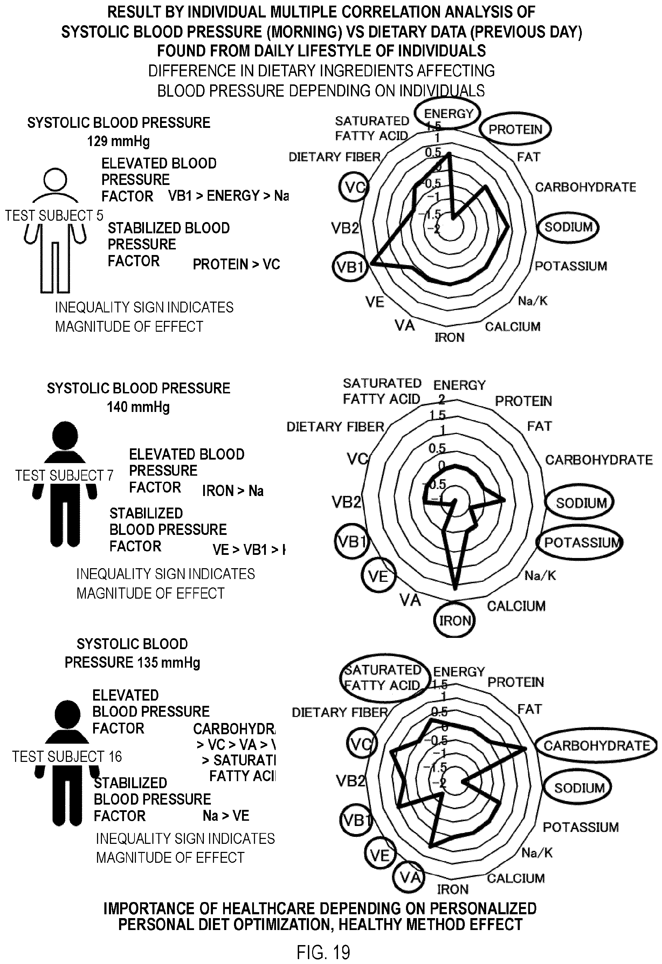

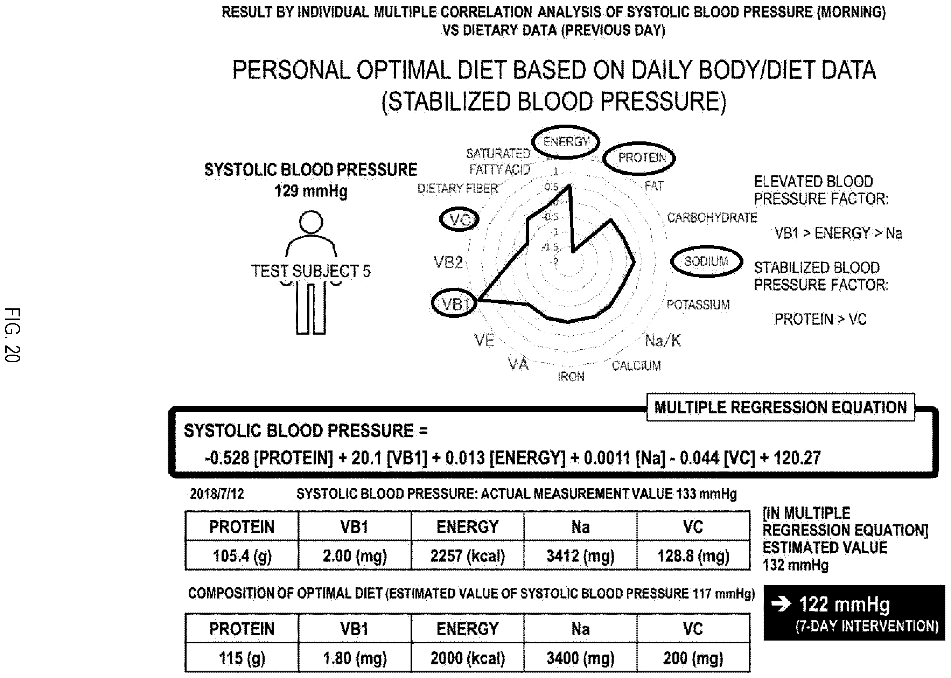

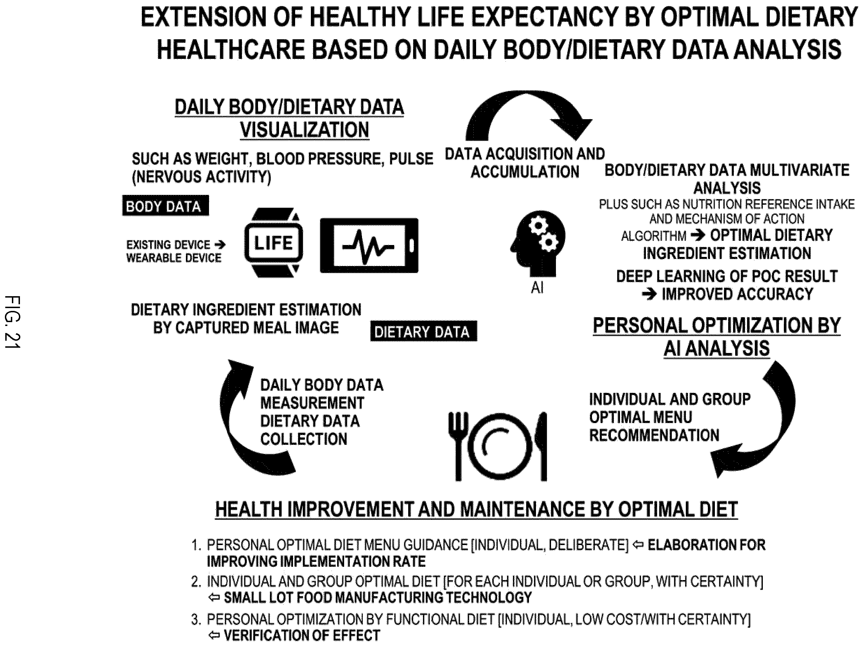

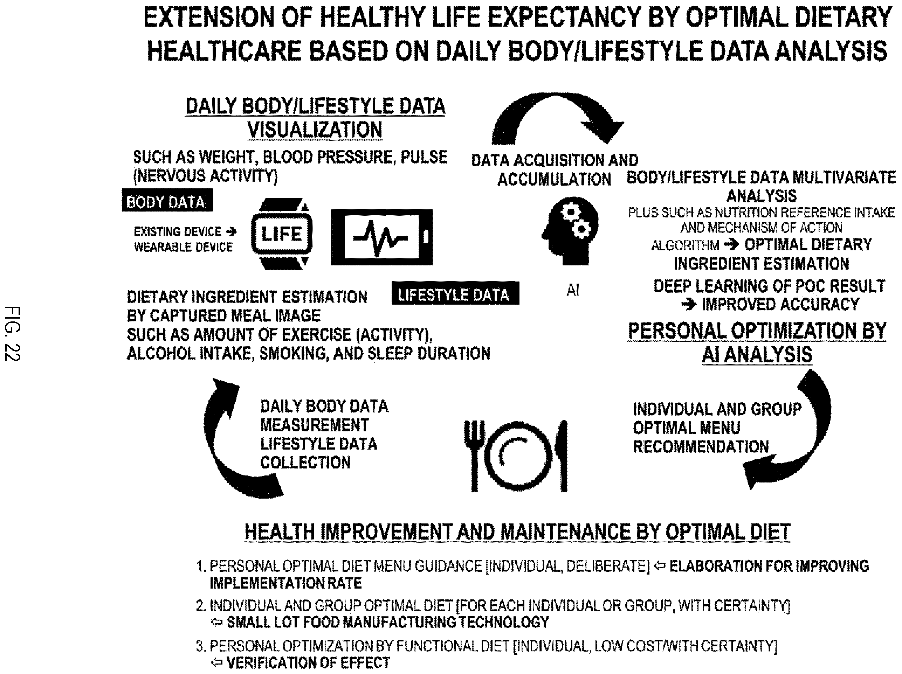

The present invention makes it possible to verify the difference in dietary ingredients that affect blood pressure among individuals found from the daily lifestyle of each individual, enabling providing the personal dietary optimization and healthy method effect only for the individual (Fig. 19). In one example, the concept of the optimal personal diet (based on nutritional values) relating to the test subject 5 in Fig. 19 is as illustrated in Fig. 20. In addition, the present invention is intended to achieve the extension of healthy life expectancy by optimal dietary healthcare based on daily body/dietary data analysis (Fig. 21) and the extension of healthy life expectancy by optimal dietary healthcare based on daily body/lifestyle data analysis (Fig. 22).

### Reference Signs List

- 1: User equipment
- 2: Recommendation apparatus
- 3: Communication network
- 4: Vital sensor
- 111: Vital data acquisition unit
- 112: Meal image capturing unit
- 113: Dietary ingredient acquisition unit
- 114: User data transmission unit
- 115: Dietary information reception unit
- 116: Dietary information display unit
- 131: Vital data storage unit
- 132: Dietary ingredient storage unit
- 211: Vital data acquisition unit
- 212: Dietary ingredient acquisition unit
- 213: Analysis processing unit
- 214: Dietary information output unit
- 215: Target value input unit
- 216: Intake determination unit
- 217: Dietary constituent input unit
- 218: Functional food selection unit
- 231: Vital data storage unit
- 232: Dietary ingredient storage unit
- 233: Model storage unit

- 234: Dietary reference intake storage unit
- 235: Functional food information storage unit

## Claims

1. A health management system comprising:
a vital data acquisition unit configured to acquire vital data of a user;
a dietary ingredient acquisition unit configured to acquire a dietary ingredient;
an analysis processing unit configured to analyze a relationship between an intake of the dietary ingredient and improvement or deterioration of the vital data; and
a dietary ingredient output unit configured to output the dietary ingredient of improving the vital data and the dietary ingredient of deteriorating the vital data.

2. The health management system according to claim 1,
wherein
the analysis processing unit estimates, in a statistical model using a measured value of the vital data as a response variable and the intake of the dietary ingredient as an explanatory variable, a coefficient of the explanatory variable by multivariate analysis.

3. The health management system according to claim 2, further comprising:
a target value input unit configured to receive an input of a target value of the vital data; and
an intake determination unit configured to determine the intake of the dietary ingredient in such a way that the vital data matches the target value.

4. The health management system according to claim 3, further comprising:
a reference value storage unit configured to store a reference value of the intake,
wherein the intake determination unit determines the intake in such a way that a deviation value from the reference value falls in a predetermined range.

5. The health management system according to any one of claims 1 to 4,
wherein
the intake determination unit determines the intake of the dietary ingredient for a plurality of the users in such a way that the vital data is closest to the target value.

6. The health management system according to any one of claims 1 to 5,
further comprising:
a menu information storage unit configured to store menu information; and
a menu creation unit configured to determine a menu including the dietary ingredient of the determined intake by referring to the menu information.

7. The health management system according to any one of claims 1 to 6,
further comprising:
a supplementary food recommendation unit configured to recommend a supplementary food including the dietary ingredient that improves the vital data in a case where the dietary ingredient is not ingested by the user.

8. The health management system according to any one of claims 1 to 7,
further comprising:
a menu information acquisition unit configured to acquire the menu information input from the user,
wherein the dietary ingredient acquisition unit specifies and acquires the dietary ingredient from the menu information.

9. The health management system according to claim 8,
wherein
the menu information includes at least foodstuff information and quantity information.

10. The health management system according to any one of claims 1 to 9,
wherein
the vital data is a result measured by a blood pressure monitor.

11. The health management system according to any one of claims 1 to 10,
further comprising:
a lifestyle information input unit configured to receive an input of lifestyle information indicating a lifestyle of the user,
wherein the analysis processing unit analyzes a relationship between the lifestyle and the intake of the dietary ingredient and improvement or deterioration of the vital data.
